# EUROPÄISCHE PATENTSCHRIFT

(11) **EP 1 490 123 B1**
(45) Veröffentlichungstag und Bekanntmachung des Hinweises auf die Patenterteilung: **06.09.2006**
(21) Anmeldenummer: 03745242.2
(22) Anmeldetag: 02.04.2003
(51) Int. Cl.: A61L 27/44, A61L 27/46, A61L 27/52, A61L 27/56

(54) **KNET- UND FORMBARE KNOCHENERSATZMASSE**
KNEADABLE AND PLIABLE BONE REPLACEMENT MATERIAL
MATERIAU DE SUBSTITUTION OSSEUSE PETRISSABLE ET FACONABLE

(30) Priorität: 03.04.2002 WO PCT/CH02/00184
(43) Veröffentlichungstag der Anmeldung: 29.12.2004
(73) Patentinhaber: Synthes GmbH, 4436 Oberdorf (CH)
(72) Erfinder: RIZZOLI, Giancarlo, CH-3012 Bern (CH); STOLL, Thierry, CH-2572 Sutz (CH); LUGINBÜHL, Reto, CH-3700 Spiez (CH); BOHNER, Marc, CH-2540 Grenchen (CH)
(74) Vertreter: Lusuardi, Werther
(86) Internationale Anmeldenummer: PCT/CH2003/000216
(87) Internationale Veröffentlichungsnummer: WO 2003/082365

(56) Entgegenhaltungen:
- EP-A- 0 416 398
- EP-A- 0 522 569
- EP-A- 1 127 581
- WO-A-93/20858
- US-A- 4 713 076
- US-A- 4 780 450
- US-A- 5 258 028
- US-A- 5 338 772

## Beschreibung

Die Erfindung betrifft eine knet- und formbare Knochenersatzmasse gemäss dem Oberbegriff des Patentanspruchs 1.

Aus dem Stand der Technik ist es bereits bekannt, Blöcke oder Granulate aus synthetisch produziertem Kalziumphosphat zur Auffüllung von Knochendefekten zu verwenden. Die Nachteile dieses Materials liegen darin, dass die Blöcke entsprechend dem zu versorgenden Knochendefekt zugeschnitten werden müssen, bzw. der Zeitaufwand bei der Applikation der losen Granulate nicht optimal ist.

Im weiteren sind einspritzbare Massen bekannt, welche allerdings im wesentlichen aus sphärischen Partikel (Kügelchen) bestehen. Solche Massen mit sphärischen Kügelchen sind leichter in den Knochen einspritzbar, weil die Kügelchen leichter aneinander vorbeigleiten. Für eine knet- und formbare Knochenersatzmasse ist dies aber nachteilig. Knet- und formbare Knochenersatzmassen sind nicht dazu bestimmt eingespritzt, sondern geknetet zu werden. Diese Masse sollte daher eher zusammenhalten, was mit sphärischen Partikeln nicht gefördert wird.

Unter dem Begriff "Partikel" soll im folgenden jeder dreidimensionale Körper, unabhängig von seinen Dimensionen verstanden werden. Insbesondere sollen darunter auch unter den Begriffen "Granulat" oder "Körner"bekannte Teilchen verstanden werden.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung oder ihrer Priorität auch tatsächlich publiziert oder öffentlich bekannt war.

Hier will die Erfindung Abhilfe schaffen. Der Erfindung liegt das Problem zugrunde, eine knetbare Knochenersatzmasse zu schaffen, welche die obengenannten Nachteile, insbesondere die Formgebung und den zeitlichen Applikationsaufwand überwindet.

Die Erfindung löst die gestellte Aufgabe mit einer knet- und formbaren Knochenersatzmasse, welche die Merkmale des Anspruchs 1 aufweist.

Damit ist der Vorteil erzielbar, dass gegenüber Knochenersatzprodukten natürlichen Ursprungs - wegen der Abwesenheit von möglichen krankheitserregenden Proteinen, Keimen, Viren oder Bakterien - die Gefahr einer Krankheitsübertragung nicht besteht.

Ein weiterer Vorteil besteht darin, dass es nicht mehr notwendig ist, die losen Keramikpartikel mühsam eines nach dem anderen an den gewünschten Einsatzort zu bringen; statt dessen kann nun mittels der knetbaren Knochenersatzmasse rasch und einfach die benötigte Gesamtmenge an Keramik-Partikeln an den Einsatzort verbracht werden. Zusätzlich fördern unrunde, insbesondere kantige Partikel die Resorption der Keramik und beschleunigen das Einwachsen von Gewebe und die Knochenheilung. Gegenüber den Massen mit annähernd sphärischen Partikeln ergibt sich der Vorteil, dass die unrunden, insbesondere kantigen Partikel den Zusammenhalt der knetbaren Masse fördern.

Als "unrund" wird jede Form der Partikel angesehen, welche im wesentlichen von der sphärischen Form abweicht. Bei einer Ausführungsform der Erfindung weisen die Keramik-Partikel eine kantige Form auf. Als "kantig" werden jene Partikel angesehen, welche einzelne Kanten aufweist, insbesondere solche, welche von blossem Auge sichtbar sind, d.h. eine Dimension von mindestens 0,1 mm aufweisen.

Gegenüber runden Partikeln ergibt sich eine Vergrösserung der Partikeloberfläche bei gleichbleibendem mittlerem Partikeldurchmesser und damit eine Erhöhung der adhäsiven Interaktion zwischen den Partikeln und dem Hydrogel. Dadurch wird die Formbarkeit der Knochenersatzmasse gewährleistet, ohne den mengenmässigen Anteil des Hydrogels erhöhen zu müssen, bzw., dessen Konzentration zu erhöhen.

Bei einer besonderen Ausführungsform weisen die Keramikpartikel ein Sphärizitätsverhältnis S = Dₘₐₓ / Dₘᵢₙ zwischen dem grössten Durchmesser Dₘₐₓ und dem kleinsten Durchmesser Dₘᵢₙ der einzelnen Partikel auf, welches grösser als 1,2 vorzugsweise grösser als 1,5 ist. Vorteilhafterweise ist der Wert von S grösser als 3, vorzugsweise grösser als 5.
Vorzugsweise weisen mindesten 60 Gew.-%, typischerweise mindesten 80 Gew.-% der Keramikpartikel eine unrunde Form auf.

Die Porengrösse der Keramik-Partikel liegt vorzugsweise im Bereich von 1 bis 500 Mikrometer. Vorzugsweise weisen wenigstens 50% der Keramik-Partikel eine Porengrösse im Bereich von 100 bis 500 Mikrometer auf.
Typischerweise haben die Keramik-Partikel einen Anteil Makroporen mit einer Grösse von 100 bis 500 Mikrometer und einen Anteil Mikroporen mit einer Grösse von 1 bis 100 Mikrometer. Dies hat den Vorteil, dass die Porengrössenverteilung optimal ist und das Durchwachsen der Poren mit autogenem Gewebe gewährleistet ist. Vorzugsweise liegt die Porengrösse im Bereich von 340 bis 450 Mikrometer.
Vorzugsweise liegt die Porosität der Keramik-Partikel im Bereich von 60 bis 90 Prozent. Dies hat den Vorteil, dass ein möglichst grosser Volumenanteil der Keramik-Partikel mit autogenem Gewebe durchwachsen werden kann.
Die Schüttdichte der Keramik-Partikel liegt vorteilhafterweise im Bereich von 0,2 g/cm³ bis 2,0 g/cm³ liegt. Typischerweise liegt die Schüttdichte der Keramik-Partikel im Bereich von 0,6 g/cm³ bis 1,0 g/cm³ , vorzugsweise im Bereich von 0,7 g/cm³ bis 0,9 g/cm³ . Bei einer Ausführungsform liegt die Schüttdichte der Keramik-Partikel im Bereich von 1,0 g/cm³ bis 2,0 g/cm³, vorzugsweise im Bereich von 0,2 g/cm³ bis 1,8 g/cm³.
Die Vorteile der höheren Schüttdichten-Bereiche sind eine höhere mechanische Stabilität, Nachteile jedoch sind langsamere Resorption und langsameres Knocheneinwachsen. Umgekehrt sind die Vorteile der tieferen Schüttdichten-Bereiche die schnellere Resorption und das bessere Knocheneinwachsen.

Die Rütteldichte der Keramik-Partikel liegt vorteilhafterweise im Bereich von 0,5 g/cm³ bis 2,5 g/cm³ liegt. Vorzugsweise liegt die Rütteldichte der Keramik-Partikel im Bereich von 0,7 g/cm³ bis 1,1 g/cm³ oder von 1,1 g/cm³ bis 2,5 g/cm³.
Die scheinbare Dichte der Knetmasse kann durch den Gebrauch von Keramik-Partikeln unterschiedlicher Korngrösse noch erhöht werden. Der interstitielle Raum (Totvolumen) zwischen den grösseren Partikeln wird mit kleineren Partikeln aufgefüllt. Durch das Interkalieren der Keramik-Partikel werden die mechanischen Eigenschaften der Knetmasse weiter verbessert.

Der mittlere Durchmesser der Keramikpartikel liegt zweckmässigerweise im Bereich von 100 bis 250 Mikrometer. Dies hat den Vorteil, dass die Knochenersatzmasse kompakt ist. Des weiteren ist das Risiko von Reizreaktionen des teilchenumgebenden Gewebes praktisch nicht vorhanden, wenn der Durchmesser der Partikel nicht kleiner als 100 Mikrometer ist.

Der mittlere Durchmesser der Keramikpartikel kann auch im Bereich von 150 bis 500 Mikrometer, beziehungsweise im Bereich von 0,5 bis 5,6 mm liegen. damit das Füllen von mittelgrossen und von grösseren Defekten effizienter gelöst werden kann.

Es können auch Keramikpartikel mit einem mittleren Durchmesser von 100 bis 250 Mikrometer mit Partikeln des mittleren Durchmessers von 250 bis 500 Mikrometer, beziehungsweise des mittleren Durchmessers von 0,5 bis 5,6 mm zusammengemischt werden. Dies hat den Vorteil, dass die Kompaktheit der Knochenersatzmasse gewährleistet ist. Das unter Verwendung von grobkörnigem Material entstehende interstitielle Porenvolumen (Poren-Totvolumen) kann damit auf ein Minimum reduziert werden. Des weiteren besteht die Möglichkeit, durch den Einsatz verschieden grosser Keramik-Partikel das Degradations-Zeitintervall der ausgehärteten Knochenersatzmasse zu beeinflussen.

Die Keramik-Partikel bestehen vorzugsweise aus einem Kalziumphosphat, typischerweise aus Beta-Trikalziumphosphat. Dies hat den Vorteil, dass eine Keramik verwendet wird, welche in ihrer stöchiometrischen Zusammensetzung weitgehend derjenigen des menschlichen Knochens entspricht. Auch ist die Degradationszeit von Beta-Trikalziumphosphat weder zu schnell noch zu langsam, als dass Hohlräume, bzw. Implantats-Rückstände im Verlauf der Degradation entstehen könnten.
Die aus Kalziumphosphat bestehenden Keramik-Partikel besitzen vorteilhafterweise ein molares Ca/P Verhältnis im Bereich von 1,0 bis 2,0 und vorzugsweise im Bereich von 1,45 bis 1,52 gekennzeichnet ist. Ein Bereich von 1,45 bis 1,49 wird besondere bevorzugt.

Das Kalziumphosphat kann aus folgender Gruppe ausgewählt werden: Dikalziumphosphatdihydrat (CaHPO₄ x 2 H₂O), Dikalziumphosphat (CaHPO₄), alpha-Trikalziumphosphat (alpha-Ca₃(PO₄)₂) , beta-Trikalziumphosphat (beta-Ca₃(PO₄)₂), kalziumdefizientes Hydroxylapatit (Ca₉(PO₄)₅(HPO₄)OH), Hydroxylapatit (Ca₁₀(PO₄)₆OH)₂), karboniertes Apatit (Ca₁₀(PO₄)₃(CO₃)₃(OH)₂), Fluorapatit (Ca₁₀(PO₄)₆(F,OH)₂), Chlorapatit (Ca₁₀(PO₄)₆(Cl,OH)₂), Whitlockit ((Ca,Mg)₃(PO₄)₂), Tetrakalziumphosphat (Ca₄(PO₄)₂O), Oxyapatit (Ca₁₀(PO₄)₆O), beta-Kalziumpyrophosphat (beta-Ca₂(P₂O₇), alpha-Kalziumpyrophosphat , gamma-Kalziumpyrophosphat, Octocalciumphosphat (Ca₈H₂(PO₄)₆ x 5 H₂O).

Die Keramik-Partikel können auch aus einem Gemisch verschiedener Kalziumphosphate bestehen. Der Vorteil eines solchen Gemisches liegt in der Steuerung der Resorptionszeit. Durch das unterschiedliche Resorptionsverhalten der Gemischkomponenten kann ein schnelleres Einwachsen vom Knochen in die Kavitäten von schneller resorbierbaren Komponenten ermöglicht werden.

Die Keramik-Partikel können auch aus Kalziumsulfat oder aus Kalziumkarbonat bestehen.

Bei einer besonderen Ausführungsform können die Keramik-Partikel aus folgender Gruppe ausgewählt werden: alpha-Kalziumsulfathemihydrat, beta-Kalziumsulfathemihydrat, Kalziumsulfatdihydrat.

Bei einer weiteren Ausführungsform können die Keramik-Partikel aus einem Gemisch von verschiedenen Kalziumphosphaten, Kalziumsulfaten und/oder Kalziumkarbonaten bestehen. Der Vorteil eines solchen Gemisches liegt in der Steuerung der Resorptionszeit. Durch das unterschiedliche Resorptionsverhalten der Gemischkomponenten kann ein schnelleres Einwachsen vom Knochen in die Kavitäten von schneller resorbierbaren Komponenten ermöglicht werden.

Die unrunden Partikel können durch Brechen oder Zerreiben von grösseren porösen Blöcken des gewünschten Materials hergestellt werden und durch entsprechende Siebung können die gewünschten Partikelgrössen gewonnen werden.

Bei einer besonderen Ausführungsform kann die Knochenersatzmasse zusätzlich noch metallische oder halbmetallische Ionenanteile enthalten. Die Vorteile solcher metallische oder halbmetallische Ionenanteile enthalten. Die Vorteile solcher Ionenanteile sind deren Einfluss auf das Resorptionsverhalten der Keramik, damit ein optimierter Ersatz der mineralischen Zusammensetzung des Knochen erreicht werden kann.

Die das Hydrogel bildende Matrix oder die zu einem Hydrogel quellbare Substanz kann aus folgenden Substanzgruppen ausgewählt sein:
a) rein synthetische Substanzen;
b) natürliche biologische Substanzen pflanzlichen Ursprungs; und/oder
c) biotechnologisch erzeugte Substanzen.
Das Hydrogel oder die zu einem Hydrogel quellbare Substanz kann auch aus einer Mischung von rein synthetischen, natürlichen biologischen oder biotechnologisch erzeugten Substanzen bestehen.

Von einem Hydrogel spricht man, wenn ein Feststoff durch eine wässerige Phase hydratisiert wird, und dabei die Viskosität der wässerigen Phase verändert und erhöht, d.h. wenn die wässerige Phase geliert oder koaguliert.

Die Hydrogel-Matrix kann aus oligomerischen oder polymerischen Anteilen bestehen oder aus einer Kombination dieser beiden. Der Knochenersatzmasse können für gegebene Indikationen zusätzlich Pharmaka als Additive beigemischt werden. Die gelierende Flüssigkeit für das Hydrogel kann Wasser, insbesondere deionisiertes Wasser und/oder ein organisches, körperverträgliches Lösungsmittel sein.

Bei einer besonderen Ausführungsform enthält das Hydrogel oder die zu einem Hydrogel quellbaren Substanzen einen der folgenden Bausteine: a) Polyaminosäuren und deren Derivate, vorzugsweise Polylysin oder Gelatine; b) Polysaccharide und deren Derivate, vorzugsweise eine Glycosaminoglycan oder Alginat; c) Polylipide, Fettsäuren und deren Derivate; d) Nucleotide und deren Derivate; oder eine Kombination der Bausteine gemäss a) bis d).

Bei einer weiteren Ausführungsform enthält das Hydrogel oder die zu einem Hydrogel quellbare Substanz einen der folgenden synthetischen Bausteine enthält: a) Polymethylenoxid oder dessen Derivate; b) Polyethylen, Polyethylenoxid oder deren Derivate; c) Polypropylen, Polypropylenoxid oder deren Derivate; d) Polyacrylate oder dessen Derivate; oder eine Kombination der Bausteine gemäss a) bis d).

Bei einer besonderen Ausführungsform besteht das Hydrogel oder die zu einem Hydrogel quellbare Substanz entweder in Form eines Glycosaminoglycan oder eines Proteoglycans oder einer Mischung dieser beiden Stoffe. Das Glycosaminoglycan kann eine Hyaluronsäure, Chondroitinsulfat, Dermatansulfat, Heparansulfat, Heparin oder Keratansulfat sein.

Die Konzentration des gebrauchsfertigen, hydratisierten Hydrogels oder der gebrauchsfertigen, hydratisierten, zu einem Hydrogel quellbaren Substanz liegt vorzugsweise im Bereich von 0,1 % bis 20,0 % liegt.
Das Molekulargewicht des Hydrogels oder einer zu einem Hydrogel quellbaren Substanz ist vorteilhafterweise grösser als 300'000 Dalton und vorzugsweise grösser als 500'000 Dalton. Bei einer weiteren Ausführungsform ist das Molekulargewicht des Hydrogels oder einer zu einem Hydrogel quellbaren Substanz grösser als 1'000'000 Dalton, vorzugsweise grösser als 1`500'000 Dalton ist. Durch ein grösseres Molekulargewicht wird eine geringere Menge an Hydrogel benötigt, um eine bestimmte Viskosität zu erzielen. Deshalb können damit sehr visköse Gele hergestellt werden mit relativ wenig Hydrogelgehalt

Bei einer besonderen Ausführungsform ist das Hydrogel eine wässerige Lösung eines Hyaluronates. Die Hyaluronsäure besteht aus Glucuronsäure und Acetylglucosamin, die das Disaccharid Hyaluronsäure aufbauen. Die Hyaluronsäure bildet infolge ihrer fadenförmigen, unverzweigten Molekülstruktur hochvisköse wässerige Lösungen.

Die wässerige Lösung des Hydrogels enthält typischerweise weniger als 99 % Wasser, vorzugsweise weniger als 98 % Wasser. Im speziellen kann die wässerige Lösung weniger als 96,5 % Wasser, vorzugsweise weniger als 95 % Wasser enthalten. Solche Konzentrationen haben den Vorteil, dass eine exzellente Formbarkeit der Knochenersatzmasse gewährleistet ist.

Das Molekulargewicht der verwendeten Hyaluronsäure ist zweckmässigerweise grösser als 1,5 x 10⁶ Dalton ist. Bei einer besonderen Ausführungsform liegt das Molekulargewicht der verwendeten Hyaluronsäure im Bereich von 0,5 x 10⁶ und 1,0 x 10⁶ Dalton.
Bei einer weiteren Ausführungsform ist das Molekulargewicht der verwendeten Hyaluronsäure kleiner als 1 x 10⁶ Dalton, vorzugsweise kleiner als 0,5 x 10⁶ Dalton.

Bei einer besonderen Ausführungsform liegt das spezifische Gewicht der kalziumhaltigen, porösen Keramik-Partikel im Bereich von 0,5 bis 1,0 g/cm³ liegt.

Bei einer weiteren Ausführungsform ist das Gewichtsverhältnis A/B zwischen dem hydratisierten Hydrogel und den kalziumhaltigen Keramik-Partikeln grösser als 0,2. Vorzugsweise liegt das Gewichtsverhältnis A/B im Bereich von 0,2 und 0,5 liegt.
Bei weiteren Ausführungsformen ist das Gewichtsverhältnis A/B im Bereich von 0,5 - 0,9 oder von 0,9 -1,3 oder von 1,3 -2,0 oder von 2 - 5 oder grösser als 5.
Die Vorteile dieser verschiedenen Bereiche für das Gewichtsverhältnis von A/B sind die unterschiedlichen Knetbarkeiten und Resorptionszeiten. Bei hohen A-Anteilen ist die Masse knetbarer, dafür schneller resorbierbar; bei hohen B-Anteilen ist die Masse weniger knetbar, dafür weniger schnell resorbierbar.

Die Erfindung und Weiterbildungen der Erfindung werden im folgenden anhand mehrerer Ausführungsbeispiele noch näher erläutert.

### Beispiel 1

1,2 g poröse und kantige Granulate von Beta-Trikalziumphosphat (β-TCP) mit einer ungefähren Grösse von 500 Mikrometer und mit einem Sphärizitätsgrad S = 3,1 wurden mit 2,0 g einer 5 %-igen wässerigen Lösung von biotechnologisch erzeugtem Natriumhyaluronat mit einem Molekulargewicht von 500 kD gemischt. Die erhaltene, knetbare Masse eignete sich hervorragend als plastisches Knochenersatzmaterial. Sie kann nach Belieben mit den Fingern geknetet werden und in eine gewünschte Form gebracht werden und dann direkt in den zu füllenden Knochendefekt gestopft werden. Die Verformbarkeit erlaubt ein optimales Auffüllen von Knochendefekten.

### Beispiel 2

Ein Gemisch aus 0,6 g porösen und kantigen Granulaten von Beta-Trikalziumphosphat (β-TCP) mit einer ungefähren Grösse von 100 Mikrometern und einem Sphärizitätsgrad S = 2,9 und 0,6 g poröse und kantige Granulate von Beta-Trikalziumphosphat (β-TCP) mit einer ungefähren Grösse von 500 Mikron und mit einem Sphärizitätsgrad S = 2,7 wurden mit 2,0 g einer 5 %-igen wässerigen Lösung von biotechnologisch erzeugtem Natriumhyaluronat mit einem Molekulargewicht von 900 kD gemischt. Die erhaltene, knetbare Masse eignete sich hervorragend als plastisches Knochenersatzmaterial.

### Beispiel 3

Ein Gemisch aus 0,3 g porösen und kantigen Granulaten von Beta-Trikalziumphosphat (β-TCP) mit einer ungefähren Grösse von 100 Mikrometern und einem Sphärizitätsgrad S = 2,4 und 0,3 g poröse und kantige Granulate von Beta-Trikalziumphosphat (β-TCP) mit einer ungefähren Grösse von 500 Mikron und mit einem Sphärizitätsgrad S = 2,3 wurden mit 1,0 g einer 10 %-igen wässerigen Lösung von biotechnologisch erzeugtem Natriumhyaluronat mit einem Molekulargewicht von 900 kD gemischt. Die erhaltene, knetbare Masse eignete sich hervorragend als plastisches Knochenersatzmaterial.

### Beispiel 4

Ein Gemisch aus 0,3 g porösen und kantigen Granulaten von Beta-Trikalziumphosphat (β-TCP) mit einer ungefähren Grösse von 100 Mikrometern und einem Sphärizitätsgrad S = 1,8 und 0,3 g poröse und kantige Granulate von Beta-Trikalziumphosphat (β-TCP) mit einer ungefähren Grösse von 500 Mikron und mit einem Sphärizitätsgrad S = 2,7 wurden mit 50 mg biotechnologisch erzeugtem Natriumhyaluronat mit einem Molekulargewicht von 900 kD vermischt. Danach wurden 0,9 g deioniertes Wasser zugegeben und während 10 Minuten gut gemischt. Die erhaltene, knetbare Masse eignete sich hervorragend als plastisches Knochenersatzmaterial.

### Beispiel 5

Ein Gemisch aus 1,65 g porösen und kantigen Granulaten von Beta-Trikalziumphosphat (β-TCP) mit einer Korngrösse von 500 bis 700 Mikrometern und einem Sphärizitätsgrad S = 2,5 und 1,65 g poröse und kantige Granulate von Beta-Trikalziumphosphat (β-TCP) mit einer Korngrösse von 125 bis 500 Mikrometer und einem Sphärizitätsgrad S = 2,9 wurden mit 3,0 g einer sterilen wässerigen 6%-igen Natriumhyaluronat-Lösung (Molekulargewicht des Natriumhyaluronats = 900 kD) unter sterilen Bedingungen mit einem Spatel vermischt. Die Masse wurde nach 30 Minuten in eine sterile tubenartige Verpackung abgefüllt. Diese sterile Knetmasse eignete sich hervorragend als plastisches Knochenersatzmaterial.

### Beispiel 6

Ein Gemisch aus 1,5 g porösen und kantigen Granulaten von Beta-Trikalziumphosphat (β-TCP) mit einer Korngrösse von 500 bis 700 Mikrometern und einem Sphärizitätsgrad S = 2,5 und 2,5 g poröse und kantige Granulate von Beta-Trikalziumphosphat (β-TCP) mit einer Korngrösse von 125 bis 500 Mikrometer und einem Sphärizitätsgrad S = 2,9 wurden mit 3,0 g einer sterilen wässerigen 8%-igen Chitosan-Lösung unter sterilen Bedingungen mit einem Spatel vermischt. Die Masse wurde nach 30 Minuten in eine sterile spritzenähnliche Verpackung abgefüllt. Die erhaltene, sterile Knetmasse eignete sich hervorragend als plastisches Knochenersatzmaterial.

### Beispiel 7

Ein Gemisch aus 1,5 g porösen und kantigen Granulaten von Beta-Trikalziumphosphat (β-TCP) mit einer Korngrösse von 500 bis 700 Mikrometern und einem Sphärizitätsgrad S = 2,5 und 1,5 g poröse und kantige Granulate von Beta-Trikalziumphosphat (β-TCP) mit einer Korngrösse von 125 bis 500 Mikrometer und einem Sphärizitätsgrad S = 2,9 wurden mit 3,0 g einer sterilen wässerigen 5%-igen rhKollagen-Lösung unter sterilen Bedingungen mit einem Spatel vermischt. Die erhaltene, sterile Knetmasse eignete sich hervorragend als plastisches Knochenersatzmaterial.

### Beispiel 8

Ein Gemisch aus 1,5 g porösen und kantigen Granulaten von Beta-Trikalziumphosphat (β-TCP) mit einer Korngrösse von 500 bis 700 Mikrometern und einem Sphärizitätsgrad S = 2,5 und 1,5 g poröse und kantige Granulate von Beta-Trikalziumphosphat (β-TCP) mit einer Korngrösse von 125 bis 500 Mikrometer und einem Sphärizitätsgrad S = 2,9 wurden mit 2,5 g einer wässerigen 5%-igen Natriumalginat-Lösung vermischt. Die erhaltene Knetmasse eignete sich hervorragend als plastisches Knochenersatzmaterial.

### Beispiel 9

3,0 g poröse und kantige Granulate von Beta-Trikalziumphosphat (β-TCP) mit einer Korngrösse von 125 bis 500 Mikrometer und einem Sphärizitätsgrad S = 2,9 wurden mit 2,5 g einer wässerigen 6,5%-igen Polyethylenglykol-Lösung (MG = 35 kD) mit einem Spatel vermischt. Die erhaltene Knetmasse eignete sich hervorragend als plastisches Knochenersatzmaterial.

### Beispiel 10

3,0 g poröse und kantige Granulate von Beta-Trikalziumphosphat (β-TCP) mit einer Korngrösse von 125 bis 500 Mikrometer und einem Sphärizitätsgrad S = 2,9 wurden mit 2,0 g einer wässerigen 4%-igen Polyethylenoxid-Lösung (MG = 511 kD) mit einem Spatel vermischt. Die erhaltene Knetmasse eignete sich hervorragend als plastisches Knochenersatzmaterial.

### Beispiel 11

3,0 g poröse und kantige Granulate von Beta-Trikalziumphosphat (β-TCP) mit einer Korngrösse von 125 bis 500 Mikrometer und einem Sphärizitätsgrad S = 2,5 wurden mit 2,2 g einer wässerigen 10%-igen Hydroxymethlyzellulose-Lösung mit einem Spatel vermischt. Die erhaltene Knetmasse eignete sich hervorragend als plastisches Knochenersatzmaterial.

### Beispiel 12

Ein Gemisch aus 1,5 g porösen und kantigen Granulaten von Beta-Trikalziumphosphat (β-TCP) mit einer Korngrösse von 500 bis 700 Mikrometern und einem Sphärizitätsgrad S = 2,5 und 1,5 g poröse und kantige Granulate von Beta-Trikalziumphosphat (β-TCP) mit einer Korngrösse von 125 bis 500 Mikrometer und einem Sphärizitätsgrad S = 2,9 wurden mit 2,5 g einer 7%-igen wässerigen Ploronic 407-Lösung vermischt. Bei Ploronic 407 handelt es sich um einen Stoff der chemischen Zusammensetzung HO(C₂H₄O)ₐ(C₃H₆O)_{b}(C₂H₄O)ₐH wobei a=101 und b=56 ist. Die erhaltene Knetmasse eignete sich hervorragend als plastisches Knochenersatzmaterial.

### Beispiel 13

Ein Gemisch aus 1,5 g porösen und kantigen Granulaten von Beta-Trikalziumphosphat (β-TCP) mit einer Korngrösse von 500 bis 700 Mikrometern und einem Sphärizitätsgrad S = 2,5 und 1,5 g poröse und kantige Granulate von Beta-Trikalziumphosphat (β-TCP) mit einer Korngrösse von 125 bis 500 Mikrometer und einem Sphärizitätsgrad S = 2,9 wurden mit 2,5 g einer wässerigen Lösung bestehend aus 0,18 g Natriumhyaluronat (MG = 1,4 Millionen Dalton) und 0,09 g Polyethylenoxid (MG = 511 kD) vermischt. Die erhaltene Knetmasse eignete sich hervorragend als plastisches Knochenersatzmaterial.

### Beispiel 14

0,2 g Natriumalginat (MG = 50-500 kD) und 1,0 g poröse und kantige Granulate von Beta-Trikalziumphosphat (β-TCP) mit einer Korngrösse von 500 bis 700 Mikrometern und einem Sphärizitätsgrad S = 2,5 und 1,5 g poröse und kantige Granulate von Beta-Trikalziumphosphat (β-TCP) mit einer Korngrösse von 125 bis 500 Mikrometer und einem Sphärizitätsgrad S = 2,9 wurden im Trockenzustand gut gemischt. Dieses Gemisch wurde in der Folge mit 2,0 g deponiertes Wasser angerührt. Daraus hat sich eine Knetmasse gebildet, welche als plastisches Knochenersatzmaterial verwendet werden konnte.

### Beispiel 15

0,18 g Natriumhyaluronat (MG = 1,1 - 1,3 Millionen Dalton) und 1,0 g poröse und kantige Granulate von Beta-Trikalziumphosphat (β-TCP) mit einer Korngrösse von 500 bis 700 Mikrometern und einem Sphärizitätsgrad S = 2,9 und 1,5 g poröse und kantige Granulate von Beta-Trikalziumphosphat (β-TCP) mit einer Korngrösse von 125 bis 500 Mikrometer und einem Sphärizitätsgrad S = 2,5 wurden im Trockenzustand gut gemischt. Dieses Gemisch wurde in der Folge mit 0,5 ml platelet-rich Plasma und 1,5 ml sterilem deionisiertem Wasser angerührt. Nach guter Durchmischung hat sich eine hervorragende plastische Knetmasse gebildet, welche als plastisches Knochenersatzmaterial verwendet werden konnte.

### Beispiel 16

0,18 g Natriumhyaluronat (MG = 1,1 - 1,3 Millionen Dalton) und 1,0 g poröse und kantige Granulate von Beta-Trikalziumphosphat (β-TCP) mit einer Korngrösse von 500 bis 700 Mikrometern und einem Sphärizitätsgrad S = 2,9 und 1,5 g poröse und kantige Granulate von Beta-Trikalziumphosphat (β-TCP) mit einer Korngrösse von 125 bis 500 Mikrometer und einem Sphärizitätsgrad S = 2,5 wurden im Trockenzustand gut gemischt. Dieses Gemisch wurde in der Folge mit 2 ml frischem Blut angerührt. Nach guter Durchmischung hat sich eine hervorragende plastische Knetmasse gebildet, welche als plastisches Knochenersatzmaterial verwendet werden konnte.

### Beispiel 17

Ein Gemisch aus 0,6 g porösen und kantigen Granulaten von Dikalziumphosphatdihydrat (CaHPO₄ x 2 H₂O) mit einer ungefähren Grösse von 100 Mikrometern und einem Sphärizitätsgrad S = 2,9 und 0,6 g poröse und kantige Granulate von Beta-Trikalziumphosphat (β-TCP) mit einer ungefähren Korngrösse von 500 Mikrometern und einem Sphärizitätsgrad S = 2,7 wurden mit 2,0 g einer 5%-igen wässerigen Lösung von biotechnologisch erzeugtem Natriumhyaluronat mit einem Molekulargewicht von 900 kD gemischt. Die erhaltene, knetbare Masse eignete sich hervorragend als plastisches Knochenersatzmaterial.

### Beispiel 18

Ein Gemisch aus 0,6 g porösen und kantigen Granulate von Dikalziumphosphat (CaHPO₄) mit einer ungefähren Grösse von 100 Mikrometern und einem Sphärizitätsgrad S = 1,5 und 0,6 g poröse und kantige Granulate von Dikalziumphosphat (CaHPO₄) mit einer ungefähren Grösse von 500 Mikrometern und einem Sphärizitätsgrad S = 2,7 wurden mit 2,0 g einer 5%-igen wässerigen Lösung von biotechnologisch erzeugtem Natriumhyaluronat mit einem Molekulargewicht von 900 kD gemischt. Die erhaltene, knetbare Masse eignete sich hervorragend als plastisches Knochenersatzmaterial.

### Beispiel 19

Ein Gemisch aus 0,3 g porösen und kantigen Granulate von kalziumdefizientem Hydroxylapatit (CDHA; Ca₉(PO₄)₅(HPO₄)OH) mit einer spezifischen Oberfläche von 55 m²/g einer ungefähren Grösse von 125 Mikrometern und einem Sphärizitätsgrad S = 1,8 und 0,3 g poröse und kantige Granulate von kalziumdefizientem Hydroxylapatit (CDHA; Ca₉(PO₄)₅(HPO₄)OH) mit einer spezifischen Oberfläche von 55 m²/g und einer ungefähren Grösse von 500 Mikrometern und einem Sphärizitätsgrad S = 2,3 wurden mit 2,7 g einer 10%-igen wässerigen Lösung von biotechnologisch erzeugtem Natriumhyaluronat mit einem Molekulargewicht von 1,2 Millionen Dalton gemischt. Die erhaltene, knetbare Masse eignete sich hervorragend als plastisches Knochenersatzmaterial.

### Beispiel 20

Ein Gemisch aus 0,3 g porösen und kantigen Granulate von kalziumdefizientem Hydroxylapatit (CDHA; Ca₉(PO₄)₅(HPO₄)OH) mit einer spezifischen Oberfläche von 102 m²/g einer ungefähren Grösse von 125 Mikrometern und einem Sphärizitätsgrad S = 1,8 und 0,3 g poröse und kantige Granulate von kalziumdefizientem Hydroxylapatit (CDHA; Ca₉(PO₄)₅(HPO₄)OH) mit einer spezifischen Oberfläche von 102 m²/g und einer ungefähren Grösse von 500 Mikrometern und einem Sphärizitätsgrad S = 2,3 wurden mit 2,7 g einer 10%-igen wässerigen Lösung von biotechnologisch erzeugtem Natriumhyaluronat mit einem Molekulargewicht von 1,2 Millionen Dalton gemischt. Die erhaltene, knetbare Masse eignete sich hervorragend als plastisches Knochenersatzmaterial.

### Beispiel 21

Ein Gemisch aus 0,3 g porösen und kantigen Granulate von Kalziumsulfathemihydrat mit einer ungefähren Grösse von 125 Mikrometern und einem Sphärizitätsgrad S = 1,8 und 0,3 g poröse und kantige Granulate von von Beta-Trikalziumphosphat (β-TCP) mit einer ungefähren Korngrösse von 500 Mikrometern und einem Sphärizitätsgrad S = 2,3 wurden mit 2,7 g einer 7%-igen wässerigen Lösung von biotechnologisch erzeugtem Natriumhyaluronat mit einem Molekulargewicht von 1,4 Millionen Dalton gemischt. Die erhaltene, knetbare Masse eignete sich hervorragend als plastisches Knochenersatzmaterial.

## Patentansprüche

1. Knet- und formbare Knochenersatzmasse, welche ein Gemisch aus
A) kalziumhaltigen Keramikpartikeln; und
B) einem Hydrogel oder einer zu einem Hydrogel quellbaren Substanz umfasst,
**dadurch gekennzeichnet, dass**
C) die Keramik-Partikel vollsynthetischen Ursprungs sind;
D) die einzelnen Keramik-Partikel eine mindestens teilweise zusammenhängende, poröse Struktur aufweisen; und
E) die Mehrzahl der Keramik-Partikel eine unrunde Form aufweisen.

2. Knochenersatzmasse nach Anspruch 1, **dadurch gekennzeichnet, dass** die Keramik-Partikel eine kantige Form aufweisen.

3. Knochenersatzmasse nach Anspruch 1 oder 2, **dadurch gekennzeichnet, dass** die Keramikpartikel ein Sphärizitätsverhältnis S = Dₘₐₓ / Dₘᵢₙ zwischen dem grössten Durchmesser Dₘₐₓ und dem kleinsten Durchmesser Dₘᵢₙ aufweist, welches grösser als 1,2, vorzugsweise grösser als 1,5 ist.

4. Knochenersatzmasse nach Anspruch 3, **dadurch gekennzeichnet, dass** das Sphärizitätsverhältnis S grösser als 3, vorzugsweise grösser als 5 ist.

5. Knochenersatzmasse nach einem der Ansprüche 1 bis 3, **dadurch gekennzeichnet, dass** mindesten 50 Gew.-%, vorzugsweise mindesten 90 Gew.-% der Keramik-Partikel eine unrunde Form aufweisen.

6. Knochenersatzmasse nach einem der Ansprüche 1 bis 5, **dadurch gekennzeichnet, dass** die Porengrösse der Keramik-Partikel im Bereich von 1 bis 500 Mikrometer liegt.

7. Knochenersatzmasse nach einem der Ansprüche 1 bis 6, **dadurch gekennzeichnet, dass** wenigstens 50 % der Keramik-Partikel eine Porengrösse im Bereich von 100 bis 500 Mikrometer aufweisen.

8. Knochenersatzmasse nach Anspruch 6, **dadurch gekennzeichnet, dass** die Porengrösse im Bereich von 1 bis 100 Mikrometer liegt.

9. Knochenersatzmasse nach Anspruch 6 oder 7, **dadurch gekennzeichnet, dass** die Porengrösse im Bereich von 340 bis 450 Mikrometer liegt.

10. Knochenersatzmasse nach einem der Ansprüche 1 bis 9, **dadurch gekennzeichnet, dass** die Porosität der Keramik-Partikel im Bereich von 60 bis 90 Prozent liegt.

11. Knochenersatzmasse nach einem der Ansprüche 1 bis 10, **dadurch gekennzeichnet, dass** die Schüttdichte der Keramik-Partikel im Bereich von 0,2 g/cm³ bis 2,0 g/cm³ liegt.

12. Knochenersatzmasse nach einem der Ansprüche 1 bis 10, **dadurch gekennzeichnet, dass** die Schüttdichte der Keramik-Partikel im Bereich von 0,6 g/cm³ bis 1,0 g/cm³ , vorzugsweise im Bereich von 0,7 g/cm³ bis 0,9 g/cm³ liegt.

13. Knochenersatzmasse nach einem der Ansprüche 1 bis 10, **dadurch gekennzeichnet, dass** die Schüttdichte der Keramik-Partikel im Bereich von 1,0 g/cm³ bis 2,0 g/cm³ , vorzugsweise im Bereich von 0,2 g/cm³ bis 1,8 g/cm³ liegt.

14. Knochenersatzmasse nach einem der Ansprüche 1 bis 13, **dadurch gekennzeichnet, dass** die Rütteldichte der Keramik-Partikel im Bereich von 0,5 g/cm³ bis 2,5 g/cm³ liegt.

15. Knochenersatzmasse nach Anspruch 14, **dadurch gekennzeichnet, dass** die Rütteldichte der Keramik-Partikel im Bereich von 0,7 g/cm³ bis 1,1 g/cm³ liegt.

16. Knochenersatzmasse nach Anspruch 14, **dadurch gekennzeichnet, dass** die Rütteldichte der Keramik-Partikel im Bereich von 1,1 g/cm³ bis 2,5 g/cm³ liegt.

17. Knochenersatzmasse nach einem der Ansprüche 1 bis 16, **dadurch gekennzeichnet, dass** der Anteil von Keramik-Partikel mit unrunder Form mindestens 60 Gew.%, vorzugsweise mindestens 80 Gew.% beträgt.

18. Knochenersatzmasse nach einem der Ansprüche 1 bis 17, **dadurch gekennzeichnet, dass** der mittlere Durchmesser der Keramik-Partikel im Bereich von 100 bis 250 Mikrometer liegt.

19. Knochenersatzmasse nach einem der Ansprüche 1 bis 17, **dadurch gekennzeichnet, dass** der mittlere Durchmesser der Keramikpartikel im Bereich von 250 bis 500 Mikrometer liegt.

20. Knochenersatzmasse nach einem der Ansprüche 1 bis 17, **dadurch gekennzeichnet, dass** der mittlere Durchmesser der Keramik-Partikel im Bereich von 0,5 bis 5,6 mm liegt.

21. Knochenersatzmasse nach einem der Ansprüche 18 bis 20, **dadurch gekennzeichnet, dass** Keramikpartikel mit einem mittlere Durchmesser von 100 bis 250 Mikrometer zusammen mit solchen des mittleren Durchmessers 250 bis 500 Mikrometer und/oder zusammen mit solchen des mittleren Durchmessers 0,5 bis 5,6 mm verwendet werden.

22. Knochenersatzmasse nach einem der Ansprüche 1 bis 21, **dadurch gekennzeichnet, dass** die Keramik-Partikel aus einem Kalziumphosphat bestehen, das durch ein molares Ca/P Verhältnis im Bereich von 1,0 bis 2,0 **gekennzeichnet** ist.

23. Knochenersatzmasse nach Anspruch 22, **dadurch gekennzeichnet, dass** die Keramik-Partikel aus einem Kalziumphosphat bestehen, das durch ein molares Ca/P Verhältnis im Bereich von 1,45 bis 1,52 **gekennzeichnet** ist.

24. Knochenersatzmasse nach Anspruch 22, **dadurch gekennzeichnet, dass** die Keramik-Partikel aus einem Kalziumphosphat bestehen, das durch ein molares Ca/P Verhältnis im Bereich von 1,45 bis 1,49 **gekennzeichnet** ist.

25. Knochenersatzmasse nach einem der Ansprüche 22 bis 24, **dadurch gekennzeichnet, dass** das Kalziumphosphat aus folgender Gruppe ausgewählt ist: Dikalziumphosphatdihydrat (CaHPO₄ x 2 H₂O), Dikalziumphosphat (CaHPO₄), alpha-Trikalziumphosphat (alpha-Ca₃(PO₄)₂), beta-Trikalziumphosphat (beta-Ca₃(PO₄)₂), kalziumdefizientes Hydroxylapatit (Ca₉(PO₄)₅(HPO₄)OH), Hydroxyapatit (Ca₁₀(PO₄)₆OH)₂), karboniertes Apatit (Ca₁₀(PO₄)₃(CO₃)₃(OH)₂), Fluorapatit (Ca₁₀(PO₄)₆(F,OH)₂), Chlorapatit (Ca₁₀(PO₄)₆(Cl,OH)₂), Whitlockit ((Ca,Mg)₃(PO₄)₂), Tetrakalziumphosphat (Ca₄(PO₄)₂O), Oxyapatit (Ca₁₀(PO₄)₆O), beta-Kalziumpyrophosphat (beta-Ca₂(P₂O₇), alpha-Kalziumpyrophosphat , gamma-Kalziumpyrophosphat, Octocalciumphosphat (Ca₈H₂(PO₄)₆ x 5 H₂O).

26. Knochenersatzmasse nach einem der Ansprüche 1 bis 25, **dadurch gekennzeichnet, dass** die Keramik-Partikel aus einem Gemisch verschiedener Kalziumphosphate bestehen.

27. Knochenersatzmasse nach einem der Ansprüche 1 bis 21, **dadurch gekennzeichnet, dass** die Keramik-Partikel aus einem Kalziumsulfat bestehen.

28. Knochenersatzmasse nach einem der Ansprüche 1 bis 21, **dadurch gekennzeichnet, dass** die Keramik-Partikel aus einem Kalziumkarbonat bestehen.

29. Knochenersatzmasse nach einem der Ansprüche 1 bis 21, **dadurch gekennzeichnet, dass** die Keramik-Partikel aus folgender Gruppe ausgewählt sind: alpha-Kalziumsulfathemihydrat, beta-Kalziumsulfathemihydrat, Kalziumsulfatdihydrat.

30. Knochenersatzmasse nach einem der Ansprüche 1 bis 21, **dadurch gekennzeichnet, dass** die Keramik-Partikel aus einem Gemisch von verschiedenen Kalziumphosphaten, Kalziumsulfaten und/oder Kalziumkarbonaten bestehen.

31. Knochenersatzmasse nach einem der Ansprüche 1 bis 21, **dadurch gekennzeichnet, dass** sie zusätzlich metallische oder halbmetallische Ionenanteile enthält.

32. Knochenersatzmasse nach einem der Ansprüche 1 bis 31 **dadurch gekennzeichnet, dass** das Hydrogel oder die zu einem Hydrogel quellbare Substanz aus rein synthetische Substanzen besteht.

33. Knochenersatzmasse nach einem der Ansprüche 1 bis 31 **dadurch gekennzeichnet, dass** das Hydrogel oder die zu einem Hydrogel quellbare Substanz aus natürlichen biologischen Substanzen, vorzugsweise pflanzlichen Ursprungs besteht.

34. Knochenersatzmasse nach einem der Ansprüche 1 bis 31, **dadurch gekennzeichnet, dass** das Hydrogel oder die zu einem Hydrogel quellbare Substanz aus einer biotechnologisch erzeugten Substanz besteht.

35. Knochenersatzmasse nach einem der Ansprüche 32 bis 34, **dadurch gekennzeichnet, dass** das Hydrogel oder die zu einem Hydrogel quellbare Substanz aus einer Mischung von rein synthetischen, natürlichen biologischen oder biotechnologisch erzeugten Substanzen besteht.

36. Knochenersatzmasse nach einem der Ansprüche 1 bis 35, **dadurch gekennzeichnet, dass** das Hydrogel oder die zu einem Hydrogel quellbare Substanz einen der folgenden Bausteine enthält: a) Polyaminosäuren und deren Derivate, vorzugsweise Polylysin oder Gelatine; b) Polysaccharide und deren Derivate, vorzugsweise eine Glycosaminoglycan oder Alginat; c) Polylipide, Fettsäuren und deren Derivate; d) Nucleotide und deren Derivate; oder eine Kombination der Bausteine gemäss a) bis d).

37. Knochenersatzmasse nach einem der Ansprüche 1 bis 35, **dadurch gekennzeichnet, dass** das Hydrogel oder die zu einem Hydrogel quellbare Substanz einen der folgenden synthetischen Bausteine enthält: a) Polymethylenoxid oder dessen Derivate; b) Polyethylen, Polyethylenoxid oder deren Derivate; c) Polypropylen, Polypropylenoxid oder deren Derivate; d) Polyacrylate oder dessen Derivate; oder eine Kombination der Bausteine gemäss a) bis d).

38. Knochenersatzmasse nach einem der Ansprüche 1 bis 37, **dadurch gekennzeichnet, dass** das Hydrogel oder die zu einem Hydrogel quellbare Substanz entweder in Form eines Glycosaminoglycan oder eines Proteoglycans oder einer Mischung dieser beiden Stoffe besteht.

39. Knochenersatzmasse nach Anspruch 38, **dadurch gekennzeichnet, dass** das Glycosaminoglycan eine Hyaluronsäure, Chondroitinsulfat, Dermatansulfat, Heparansulfat, Heparin oder Keratansulfat ist.

40. Knochenersatzmasse nach einem der Ansprüche 1 bis 39, **dadurch gekennzeichnet, dass** die Konzentration des gebrauchsfertigen, hydratisierten Hydrogels oder der gebrauchsfertigen, hydratisierten, zu einem Hydrogel quellbaren Substanz im Bereich von 0,1 % bis 20,0 % liegt.

41. Knochenersatzmasse nach einem der Ansprüche 1 bis 40, **dadurch gekennzeichnet, dass** das Molekulargewicht des Hydrogels oder einer zu einem Hydrogel quellbaren Substanz grösser als 300'000 Dalton, vorzugsweise grösser als 500'000 Dalton ist.

42. Knochenersatzmasse nach Anspruch 41, **dadurch gekennzeichnet, dass** das Molekulargewicht des Hydrogels oder einer zu einem Hydrogel quellbaren Substanz grösser als 1'000'000 Dalton, vorzugsweise grösser als 1'500'000 Dalton ist.

43. Knochenersatzmasse nach einem der Ansprüche 1 bis 42, **dadurch gekennzeichnet, dass** das Hydrogel eine wässerige Lösung eines Hyaluronates ist.

44. Knochenersatzmasse nach Anspruch 43, **dadurch gekennzeichnet, dass** die wässerige Lösung des Hydrogels weniger als 99 % Wasser, vorzugsweise weniger als 98 % Wasser enthält.

45. Knochenersatzmasse nach Anspruch 43, **dadurch gekennzeichnet, dass** die wässerige Lösung weniger als 96,5 % Wasser, vorzugsweise weniger als 95 % Wasser enthält.

46. Knochenersatzmasse nach einem der Ansprüche 43 bis 45, **dadurch gekennzeichnet, dass** das Molekulargewicht der verwendeten Hyaluronsäure grösser als 1,5 x 10⁶ Dalton ist.

47. Knochenersatzmasse nach einem der Ansprüche 43 bis 45, **dadurch gekennzeichnet, dass** das Molekulargewicht der verwendeten Hyaluronsäure im Bereich von 0,5 x 10⁶ und 1,0 x 10⁶ Dalton ist.

48. Knochenersatzmasse nach einem der Ansprüche 43 bis 45, **dadurch gekennzeichnet, dass** das Molekulargewicht der verwendeten Hyaluronsäure kleiner als 1 x 10⁶ Dalton, vorzugsweise kleiner als 0,5 x 10⁶ Dalton ist.

49. Knochenersatzmasse nach einem der Ansprüche 1 bis 48, **dadurch gekennzeichnet, dass** das spezifische Gewicht der kalziumhaltigen, porösen Keramik-Partikel im Bereich von 0,5 bis 1,0 g/cm³ liegt.

50. Knochenersatzmasse nach einem der Ansprüche 1 bis 49, **dadurch gekennzeichnet, dass** das Gewichtsverhältnis A/B zwischen dem hydratisierten Hydrogel und den kalziumhaltigen Keramik-Partikeln grösser als 0,2 ist.

51. Knochenersatzmasse nach Anspruch 50, **dadurch gekennzeichnet, dass** das Gewichtsverhältnis A/B im Bereich von 0,2 und 0,5 liegt.

52. Knochenersatzmasse nach Anspruch 50, **dadurch gekennzeichnet, dass** das Gewichtsverhältnis A/B im Bereich von 0,5 und 0,9 liegt.

53. Knochenersatzmasse nach Anspruch 50, **dadurch gekennzeichnet, dass** das Gewichtsverhältnis A/B im Bereich von 0,9 und 1,3 liegt.

54. Knochenersatzmasse nach Anspruch 50, **dadurch gekennzeichnet, dass** das Gewichtsverhältnis A/B im Bereich von 1,3 und 2,0 liegt.

55. Knochenersatzmasse nach Anspruch 50, **dadurch gekennzeichnet, dass** das Gewichtsverhältnis A/B im Bereich von 2 und 5 liegt.

56. Knochenersatzmasse nach Anspruch 50, **dadurch gekennzeichnet, dass** das Gewichtsverhältnis A/B grösser als 5 ist.

## Claims

1. Kneadable and moldable bone-replacement material which consists of a mixture of
A) calcium-containing ceramic particles; and
B) a hydrogel or a substance which can be swelled into a hydrogel,
**characterised in that**
C) the ceramic particles are of fully synthetic origin;
D) the individual ceramic particles have at least a partially cohesive, porous structure; and
E) the majority of the ceramic particles have a non-spheric shape.

2. Bone-replacement material in accordance with claim 1, **characterised in that** the ceramic particles have an angular shape.

3. Bone-replacement material in accordance with claim 1 or 2, **characterised in that** the ceramic particles have a sphericity relationship S = Dmax/Dmin between the largest diameter Dmax and the smalles diameter Dmin which is larger than 1.2 and preferably larger than 1.5.

4. Bone-replacement material in accordance with claim 3, **characterised in that** the sphericity relationship S is larger than 3 and preferably larger than 5.

5. Bone-replacement material in accordance with one of the claims 1 - 3, **characterised in that** at least 50% and preferably at least 90% of the ceramic particles have a non-spheric shape.

6. Bone-replacement material in accordance with one of the claims 1 - 5, **characterised in that** the pore size of the ceramic particles is between 1 and 500 micrometers.

7. Bone-replacement material in accordance with one of the claims 1 - 6, **characterised in that** at least 50% of the ceramic particles have a pore size between 100 and 500 micrometers.

8. Bone-replacement material in accordance with claim 7, **characterised in that** the pore size is between 1 and 100 micrometers.

9. Bone-replacement material in accordance with claim 8, **characterised in that** the pore size is between 340 and 450 micrometers.

10. Bone-replacement material in accordance with one of the claims 1 **-** 9, **characterised in that** the porosity of the ceramic particles is between 60 and 90 percent.

11. Bone-replacement material in accordance with one of the claims 1 - 10, **characterised in that** the bulk density of the ceramic particles is between 0.2 g/ccm and 2.0 g/ccm.

12. Bone-replacement material in accordance with one of the claims 1 - 10, **characterised in that** the bulk density of the ceramic particles is between 0.6 g/ccm and 1.0 g/ccm and preferably between 0.7 g/ccm and 0.9 g/ccm.

13. Bone-replacement material in accordance with one of the claims 1 - 10, **characterised in that** the bulk density of the ceramic particles is between 1.0 g/ccm and 2.0 g/ccm and preferably between 0.2 g/ccm and 1.8 g/ccm.

14. Bone-replacement material in accordance with one of the claims 1 - 13, **characterised in that** the jarring density of the ceramic particles is between 0.5 g/ccm and 2.5 g/ccm.

15. Bone-replacement material in accordance with claim 14, **characterised in that** the jarring density of the ceramic particles is between 0.7 g/ccm and 1.1 g/ccm.

16. Bone-replacement material in accordance with claim 14, **characterised in that** the jarring density of the ceramic particles is between 1.1 g/ccm and 2.5 g/ccm.

17. Bone-replacement material in accordance with one of the claims 1 - 16, **characterised in that** the share of ceramic particles of non-spheric shape is at least 60% and preferably at least 80%.

18. Bone-replacement material in accordance with one of the claims 1 - 17, **characterised in that** the average diameter of the ceramic particles is between 100 and 250 micrometers.

19. Bone-replacement material in accordance with one of the claims 1 - 17, **characterised in that** the average diameter of the ceramic particles is between 250 and 500 micrometers.

20. Bone-replacement material in accordance with one of the claims 1 - 17, **characterised in that** the average diameter of the ceramic particles is between 0.5 and 5.6 mm.

21. Bone-replacement material in accordance with one of the claims 18 - 20, **characterised in that** ceramic particles with an average diameter of 100 to 250 micrometers are used together with those having an average diameter of 250 to 500 micrometers and/or together with those having an average diameter of 0.5 to 5.6 mm.

22. Bone-replacement material in accordance with one of the claims 1 - 21, **characterised in that** the ceramic particles consist of a calcium-phosphate which is **characterised by** a molar Ca/P relationship between 1.0 and 2.0.

23. Bone-replacement material in accordance with claim 22, **characterised in that** the ceramic particles consist of a calcium-phosphate which is **characterised by** a molar Ca/P relationship between 1.45 and 1.52.

24. Bone-replacement material in accordance with claim 22, **characterised in that** the ceramic particles consist of a calcium-phosphate which is **characterised by** a molar Ca/P relationship between 1.45 and 1.49.

25. Bone-replacement material in accordance with one of the claims 22- 24, **characterised in that** the calcium phosphate is selected from the following group: Dicalcium-phosphate-dihydrate (CaHPO₄ x 2 H₂O), dicalcium-phosphate (CaHPO₄), alpha-tricalcium-phosphate (alpha-Ca₃(PO₄)₂), beta-tricalcium-phosphate (beta-Ca₃(PO₄)₂), calcium-deficient hydro-xylapatite (Ca₉(PO₄)₅(HPO₄)OH), hydro-xylapatite (Ca₁₀(PO₄)₆OH)₂), carbonated apatite (Ca₁₀(PO₄)₃(CO₃)₃(OH)₂), flouride-apatite (Ca₁₀(PO₄)₆(F,OH)₂), chloride-apatite (Ca₁₀(PO₄)₆(Cl,OH)₂), whitlockite ((Ca,Mg)₃(PO₄)₂), tetracalcium-phosphate (Ca₄(PO₄)₂O), oxyapatite (Ca₁₀(PO₄)₆O), beta-calcium-pyrophosphate (beta-Ca₂(P₂O₇), alpha-calcium-pyrophosphate, gamma-calcium-pyrophosphate, octo-calcium-phosphate (Ca₈H₂(PO₄)₆ x 5 H₂O).

26. Bone-replacement material in accordance with one of the claims 1 - 25, **characterised in that** the ceramic particles consist of a mixture of different calcium-phosphates.

27. Bone-replacement material in accordance with one of the claims 1 - 21, **characterised in that** the ceramic particles consist of a calcium-sulfate.

28. Bone-replacement material in accordance with one of the claims 1 - 21, **characterised in that** the ceramic particles consist of a calcium-carbonate.

29. Bone-replacement material in accordance with one of the claims 1 - 21, **characterised in that** the ceramic particles are selected from the following group: alpha-calcium-sulfate-hemihydrate, beta-calcium-sulfate-hemihydrate, calcium-sulfate-dihydrate.

30. Bone-replacement material in accordance with one of the claims 1-21, **characterised in that** the ceramic particles consist of a mixture of different calcium-phosphates, calcium-sulfates and/or calcium-carbonates.

31. Bone-replacement material in accordance with one of the claims 1 - 21, **characterised in that** it contains metallic or semi-metallic ion shares as additives.

32. Bone-replacement material in accordance with one of the claims 1 - 31, **characterised in that** the hydrogel or the substance which can be swelled into a hydrogel consists of fully synthetic substances.

33. Bone-replacement material in accordance with one of the claims 1 - 31, **characterised in that** the hydrogel or the substance which can be swelled into a hydrogel consists of natural biological substances, preferably of plant origin.

34. Bone-replacement material in accordance with one of the claims 1 - 31, **characterised in that** the hydrogel or the substance which can be swelled into a hydrogel consists of a biotechnologically generated substance.

35. Bone-replacement material in accordance with one of the claims 32 - 34, **characterised in that** the hydrogel or the substance which can be swelled into a hydrogel consists of a mixture of fully synthetic, natural biological or biotechnologically generated substances.

36. Bone-replacement material in accordance with one of the claims 1 - 35, **characterised in that** the hydrogel or the substance which can be swelled into a hydrogel contains one of the following components: a) polyamino-acids or their derivatives, preferably polylysin or gelatin; b) polysaccharides and their derivatives, preferably glycosaminoglycane or alginate; c) polylipides, fatty acids and their derivatives; d) nucleotides and their derivatives; or a combination of the components as listed in a) through d).

37. Bone-replacement material in accordance with one of the claims 1 - 35, **characterised in that** the hydrogel or the substance which can be swelled into a hydrogel contains one of the following components: a) polymethylenoxide or its derivatives; b) polyethylene, polyethylenoxide or their derivatives; c) polypropylene, polypropylenoxide or their derivatives; d) polyacrylate or its derivatives; or a combination of the components as listed in a) through d).

38. Bone-replacement material in accordance with one of the claims 1 - 37, **characterised in that** the hydrogel or the substance which can be swelled into a hydrogel consists of either a glycosaminoglycane or a proteoglycane or a mixture of those two substances.

39. Bone-replacement material in accordance with claim 38, **characterised in that** the glycosaminoglycane is a hyaluron-acid, chondroitinsulfate, dermatansulfate, heparansulfate, heparine or keratansulfate.

40. Bone-replacement material in accordance with one of the claims 1 - 39, **characterised in that** the concentration of the ready-to-use, hydrated hydrogel or the ready-to-use, hydrated substance which can be swellen into a hydrogel is between 0.1 % and 20.0%.

41. Bone-replacement material in accordance with one of the claims 1 - 40, **characterised in that** the molecular weight of the hydrogel or the substance which can be swelled into a hydrogel is larger than 300'000 Dalton and preferably larger than 500'000 Dalton.

42. Bone-replacement material in accordance with claim 41, **characterised in that** the molecular weight of the hydrogel or the substance which can be swelled into a hydrogel is larger than 1'000'000 Dalton and preferably larger than 1'500'000 Dalton.

43. Bone-replacement material in accordance with one of the claims 1 - 42, **characterised in that** the hydrogel is a liquid solution of a hyaluronate.

44. Bone-replacement material in accordance with claim 43, **characterised in that** the liquid solution of the hydrogel contains less than 99% water and preferably less than 98% water.

45. Bone-replacement material in accordance with claim 43, **characterised in that** the liquid solution of the hydrogel contains less than 96.5% water and preferably less than 95% water.

46. Bone-replacement material in accordance with one of the claims 43 - 45, **characterised in that** the molecular weight of the hyaluron-acid used is larger than 1.5 x 10↑6 Dalton.

47. Bone-replacement material in accordance with one of the claims 43 - 45, **characterised in that** the molecular weight of the hyaluron-acid used is between 0.5 x 10↑6 and 1.0 x 10↑6 Dalton.

48. Bone-replacement material in accordance with one of the claims 43 - 45, **characterised in that** the molecular weight of the hyaluron-acid used is smaller than 1 x 10↑6 and preferably smaller than 0.5 x 10↑6 Dalton.

49. Bone-replacement material in accordance with one of the claims 1 - 48, **characterised in that** the specific gravity of the calcium-containing, porous ceramic particles is between 0.5 and 1.0 g/ccm.

50. Bone-replacement material in accordance with one of the claims 1 - 49, **characterised in that** the weight relationship A/B between the hydrated hydrogel and the calcium-containing ceramic particles is larger than 0.2.

51. Bone-replacement material in accordance with claim 50, **characterised in that** the weight relationship A/B is between 0.2 and 0.5.

52. Bone-replacement material in accordance with claim 50, **characterised in that** the weight relationship A/B is between 0.5 and 0.9.

53. Bone-replacement material in accordance with claim 50, **characterised in that** the weight relationship A/B is between 0.9 and 1.3.

54. Bone-replacement material in accordance with claim 50, **characterised in that** the weight relationship A/B is between 1.3 and 2.0.

55. Bone-replacement material in accordance with claim 50, **characterised in that** the weight relationship A/B is between 2 and 5.

56. Bone-replacement material in accordance with claim 50, **characterised in that** the weight relationship A/B is larger than 5.

## Revendications

1. Matériau de substitution osseuse pétrissable et façonnable, comprenant un mélange composé :
A) de particules de céramique contenant du calcium ; et
B) d'un hydrogel ou d'une substance gonflable pouvant former un hydrogel, **caractérisé en ce que**
C) les particules de céramique sont entièrement d'origine synthétique ;
D) les particules de céramique individuelles présentent une structure poreuse au moins partiellement interconnectée ; et
E) la plupart des particules de céramique ne présentent pas une forme ronde.

2. Matériau de substitution osseuse selon la revendication 1, **caractérisé en ce que** les particules de céramique présentent une forme polygonale.

3. Matériau de substitution osseuse selon la revendication 1 ou 2, **caractérisé en ce que** les particules de céramique présentent un rapport de sphéricité S = Dₘₐₓ/Dₘᵢₙ du diamètre le plus grand Dₘₐₓ au diamètre le plus petit Dₘᵢₙ supérieur à 1,2, de préférence supérieur à 1,5.

4. Matériau de substitution osseuse selon la revendication 3, **caractérisé en ce que** le rapport de sphéricité S est supérieur à 3, de préférence supérieur à 5.

5. Matériau de substitution osseuse selon l'une quelconque des revendications 1 à 3, **caractérisé en ce qu'**au moins 50% en poids, de préférence au moins 90% en poids des particules de céramique ne présentent pas une forme ronde.

6. Matériau de substitution osseuse selon l'une quelconque des revendications 1 à 5, **caractérisé en ce que** la taille de pores des particules de céramique est dans la gamme de 1 à 500 micromètres.

7. Matériau de substitution osseuse selon l'une quelconque des revendications 1 à 6, **caractérisé en ce qu'**au moins 50% des particules de céramique présentent une taille de pores dans la gamme de 100 à 500 micromètres.

8. Matériau de substitution osseuse selon la revendication 6, **caractérisé en ce que** la taille des pores est dans la gamme de 1 à 100 micromètres.

9. Matériau de substitution osseuse selon la revendication 6 ou 7, **caractérisé en ce que** la taille des pores est dans la gamme de 340 à 450 micromètres.

10. Matériau de substitution osseuse selon l'une quelconque des revendications 1 à 9, **caractérisé en ce que** la porosité des particules de céramique est dans la gamme de 60 à 90 pour cent.

11. Matériau de substitution osseuse selon l'une quelconque des revendications 1 à 10, **caractérisé en ce que** la masse volumique apparente des particules de céramique est dans la gamme de 0,2 g/cm³ à 2,0 g/cm³.

12. Matériau de substitution osseuse selon l'une quelconque des revendications 1 à 10, **caractérisé en ce que** la masse volumique apparente des particules de céramique est dans la gamme de 0,6 g/cm³ à 1,0 g/cm³, de préférence dans la gamme de 0,7 g/cm³ à 0,9 g/cm³.

13. Matériau de substitution osseuse selon l'une quelconque des revendications 1 à 10, **caractérisé en ce que** la masse volumique apparente des particules de céramique est dans la gamme de 1,0 g/cm³ à 2,0 g/cm³, de préférence dans la gamme de 0,2 g/cm³ à 1,8 g/cm³.

14. Matériau de substitution osseuse selon l'une quelconque des revendications 1 à 13, **caractérisé en ce que** la masse volumique apparente des particules de céramique après tassement est dans la gamme de 0,5 g/cm³ à 2,5 g/cm³.

15. Matériau de substitution osseuse selon la revendication 14, **caractérisé en ce que** la masse volumique apparente des particules de céramique après tassement est dans la gamme de 0,7 g/cm³ à 1,1 g/cm³.

16. Matériau de substitution osseuse selon la revendication 14, **caractérisé en ce que** la masse volumique apparente des particules de céramique après tassement est dans la gamme de 1,1 g/cm³ à 2,5 g/cm³.

17. Matériau de substitution osseuse selon l'une quelconque des revendications 1 à 16, **caractérisé en ce que** la proportion de particules de céramique n' ayant pas une forme ronde s'élève à au moins 60% en poids, de préférence à au moins 80% en poids.

18. Matériau de substitution osseuse selon l'une quelconque des revendications 1 à 17, **caractérisé en ce que** le diamètre moyen des particules de céramique est dans la gamme de 100 à 250 micromètres.

19. Matériau de substitution osseuse selon l'une quelconque des revendications 1 à 17, **caractérisé en ce que** le diamètre moyen des particules de céramique est dans la gamme de 250 à 500 micromètres.

20. Matériau de substitution osseuse selon l'une quelconque des revendications 1 à 17, **caractérisé en ce que** le diamètre moyen des particules de céramique est dans la gamme de 0,5 à 5,6 micromètres.

21. Matériau de substitution osseuse selon l'une quelconque des revendications 18 à 20, **caractérisé en ce que** les particules de céramique ayant un diamètre moyen de 100 à 250 micromètres sont mises en oeuvre conjointement avec celles d'un diamètre moyen de 250 à 500 micromètres et/ou conjointement avec celles d'un diamètre moyen de 0,5 à 5,6 mm.

22. Matériau de substitution osseuse selon l'une quelconque des revendications 1 à 21, **caractérisé en ce que** les particules de céramique se composent d'un phosphate de calcium, lequel est **caractérisé par** un rapport molaire Ca/P dans la gamme de 1,0 à 2,0.

23. Matériau de substitution osseuse selon la revendication 22, **caractérisé en ce que** les particules de céramique se composent d'un phosphate de calcium, lequel est **caractérisé par** un rapport molaire Ca/P dans la gamme de 1,45 à 1,52.

24. Matériau de substitution osseuse selon la revendication 22, **caractérisé en ce que** les particules de céramique se composent d'un phosphate de calcium, lequel est **caractérisé par** un rapport molaire Ca/P dans la gamme de 1,45 à 1,49.

25. Matériau de substitution osseuse selon l'une quelconque des revendications 22 à 24, **caractérisé en ce que** le phosphate de calcium est choisi dans le groupe suivant : le diphosphate de calcium dihydraté (CaHPO₄ x 2 H₂O), le diphosphate de calcium (CaHPO₄), l'alpha-triphosphate de calcium (alpha-Ca₃(PO₄)₂), le bêta-triphosphate de calcium (bêta-Ca₃(PO₄)₂), l'hydroxyapatite déficiente en calcium (Ca₉(PO₄)₅(HPO₄)OH), l'hydroxyapatite (Ca₁₀(PO₄)₆OH)₂), l'apatite carbonatée (Ca₁₀(PO₄)₃(CO₃)₃(OH)₂), l'apatite de fluor (Ca₁₀(PO₄)₆(F,OH)₂), l'apatite de chlore (Ca₁₀(PO₄)₆(Cl,OH)₂), la whitlockite ((Ca,Mg)₃(PO₄)₂), le tétraphosphate de calcium (Ca₄(PO₄)₂O), l'oxyapatite (Ca₁₀(PO₄)₆O), le bêta-pyrophosphate de calcium (bêta-Ca₂(P2O₇)), l'alpha-pyrophosphate de calcium, le gamma-pyrophosphate de calcium, l'octophosphate de calcium (Ca₈H₂(PO₄)₆ x 5 H₂O).

26. Matériau de substitution osseuse selon l'une quelconque des revendications 1 à 25, **caractérisé en ce que** les particules de céramique sont composées d'un mélange de différents phosphates de calcium.

27. Matériau de substitution osseuse selon l'une quelconque des revendications 1 à 21, **caractérisé en ce que** les particules de céramique sont composées d'un sulfate de calcium.

28. Matériau de substitution osseuse selon l'une quelconque des revendications 1 à 21, **caractérisé en ce que** les particules de céramique sont composées d'un carbonate de calcium.

29. Matériau de substitution osseuse selon l'une quelconque des revendications 1 à 21, **caractérisé en ce que** les particules de céramique sont choisies dans le groupe suivant : l'alpha-sulfate de calcium hémihydraté, le bêta-sulfate de calcium hémihydraté, le sulfate de calcium dihydraté.

30. Matériau de substitution osseuse selon l'une quelconque des revendications 1 à 21, **caractérisé en ce que** les particules de céramique sont composées d'un mélange de différents phosphates, sulfates et/ou carbonates de calcium.

31. Matériau de substitution osseuse selon l'une quelconque des revendications 1 à 21, **caractérisé en ce qu'**il contient en plus des fractions ioniques métalliques ou semimétalliques.

32. Matériau de substitution osseuse selon l'une quelconque des revendications 1 à 31, **caractérisé en ce que** l'hydrogel ou la substance gonflable pouvant former un hydrogel est composé de substances purement synthétiques.

33. Matériau de substitution osseuse selon l'une quelconque des revendications 1 à 31, **caractérisé en ce que** l'hydrogel ou la substance gonflable pouvant former un hydrogel est composé de substances biologiques naturelles, de préférence d'origine végétale.

34. Matériau de substitution osseuse selon l'une quelconque des revendications 1 à 31, **caractérisé en ce que** l'hydrogel ou la substance gonflable pouvant former un hydrogel est composé d'une substance produite par des procédés biotechnologiques.

35. Matériau de substitution osseuse selon l'une quelconque des revendications 32 à 34, **caractérisé en ce que** l'hydrogel ou la substance gonflable pouvant former un hydrogel est composé d'un mélange de substances purement synthétiques, de substances biologiques naturelles ou de substances produites par des procédés biotechnologiques.

36. Matériau de substitution osseuse selon l'une quelconque des revendications 1 à 35, **caractérisé en ce que** l'hydrogel ou la substance gonflable pouvant former un hydrogel contient l'un des éléments suivants : a) des poly(acides aminés) et leurs dérivés, de préférence la polylysine ou la gélatine ; b) des polysaccharides et leurs dérivés, de préférence un glycosaminoglycane ou un alginate ; c) des polylipides, des acides gras et leurs dérivés ; d) des nucléotides et leurs dérivés ; ou une combinaison des éléments selon a) à d).

37. Matériau de substitution osseuse selon l'une quelconque des revendications 1 à 35, **caractérisé en ce que** l'hydrogel ou la substance gonflable pouvant former un hydrogel contient l'un des éléments synthétiques suivants : a) le polyoxyméthylène ou ses dérivés ; b) le polyéthylène, le poly(oxyde d'éthylène) ou leurs dérivés ; c) le polypropylène, le poly(oxyde de propylène) ou leurs dérivés ; d) le polyacrylate ou ses dérivés ; ou une combinaison des éléments selon a) à d).

38. Matériau de substitution osseuse selon l'une quelconque des revendications 1 à 37, **caractérisé en ce que** l'hydrogel ou la substance gonflable pouvant former un hydrogel se présente sous la forme d'un glycosaminoglycane ou d'un protéoglycane ou d'un mélange de ces deux substances.

39. Matériau de substitution osseuse selon la revendication 38, **caractérisé en ce que** le glycosaminoglycane est un acide hyaluronique, le sulfate de chondroïtine, le sulfate de dermatane, le sulfate d'héparane, l'héparine ou le sulfate de kératane.

40. Matériau de substitution osseuse selon l'une quelconque des revendications 1 à 39, **caractérisé en ce que** la concentration de l'hydrogel hydraté prêt à l'emploi ou de la substance gonflable pouvant former un hydrogel, hydratée, prête à l'emploi est dans la gamme de 0,1% à 20,0%.

41. Matériau de substitution osseuse selon l'une quelconque des revendications 1 à 40, **caractérisé en ce que** la masse moléculaire de l'hydrogel ou d'une substance gonflable pouvant former un hydrogel est supérieure à 300 000 daltons, de préférence supérieure à 500 000 daltons.

42. Matériau de substitution osseuse selon la revendication 41, **caractérisé en ce que** la masse moléculaire de l'hydrogel ou d'une substance gonflable pouvant former un hydrogel est supérieure à 1 000 000 daltons, de préférence supérieure à 1 500 000 daltons.

43. Matériau de substitution osseuse selon l'une quelconque des revendications 1 à 42, **caractérisé en ce que** l'hydrogel est une solution aqueuse d'un hyaluronate.

44. Matériau de substitution osseuse selon la revendication 43, **caractérisé en ce que** la solution aqueuse de l'hydrogel contient moins de 99% d'eau, de préférence moins de 98% d'eau.

45. Matériau de substitution osseuse selon la revendication 43, **caractérisé en ce que** la solution aqueuse contient moins de 96,5% d'eau, de préférence moins de 95% d'eau.

46. Matériau de substitution osseuse selon l'une quelconque des revendications 43 à 45, **caractérisé en ce que** la masse moléculaire de l'acide hyaluronique utilisé est supérieure à 1,5 x 10⁶ daltons.

47. Matériau de substitution osseuse selon l'une quelconque des revendications 43 à 45, **caractérisé en ce que** la masse moléculaire de l'acide hyaluronique utilisé est dans la gamme de 0,5 x 10⁶ à 1,0 x 10⁶ daltons.

48. Matériau de substitution osseuse selon l'une quelconque des revendications 43 à 45, **caractérisé en ce que** la masse moléculaire de l'acide hyaluronique utilisé est inférieure à 1 x 10⁶ daltons, de préférence inférieure à 0,5 x 10⁶ daltons.

49. Matériau de substitution osseuse selon l'une quelconque des revendications 1 à 48, **caractérisé en ce que** la masse volumique des particules de céramique poreuses contenant du calcium est dans la gamme de 0,5 à 1,0 g/cm³.

50. Matériau de substitution osseuse selon l'une quelconque des revendications 1 à 49, **caractérisé en ce que** le rapport pondéral A/B de l'hydrogel hydraté aux particules de céramique contenant du calcium est supérieur à 0,2.

51. Matériau de substitution osseuse selon la revendication 50, **caractérisé en ce que** le rapport pondéral A/B est dans la gamme de 0,2 à 0,5.

52. Matériau de substitution osseuse selon la revendication 50, **caractérisé en ce que** le rapport pondéral A/B est dans la gamme de 0,5 à 0,9.

53. Matériau de substitution osseuse selon la revendication 50, **caractérisé en ce que** le rapport pondéral A/B est dans la gamme de 0,9 à 1,3.

54. Matériau de substitution osseuse selon la revendication 50, **caractérisé en ce que** le rapport pondéral A/B est dans la gamme de 1,3 à 2,0.

55. Matériau de substitution osseuse selon la revendication 50, **caractérisé en ce que** le rapport pondéral A/B est dans la gamme de 2 à 5.

56. Matériau de substitution osseuse selon la revendication 50, **caractérisé en ce que** le rapport pondéral A/B est supérieur à 5.
